# EUROPÄISCHE PATENTSCHRIFT

(11) **EP 2 536 678 B1**
(45) Veröffentlichungstag und Bekanntmachung des Hinweises auf die Patenterteilung: **23.11.2016**
(21) Anmeldenummer: 11700256.8
(22) Anmeldetag: 11.01.2011
(51) Int. Cl.: C07C 45/68, C07C 49/798, C11D 3/39, C11D 3/20, C11D 3/50, A61K 8/35, A61Q 17/04, A61Q 19/10, A61Q 5/02, C11B 9/00

(54) **PHOTOLABILE DUFTSPEICHERSTOFFE**
PHOTOLABILE PRO-FRAGRANCES
SUBSTANCES PHOTOLABILES RENFERMANT UN PARFUM

(30) Priorität: 17.02.2010 DE 102010002007
(43) Veröffentlichungstag der Anmeldung: 26.12.2012
(73) Patentinhaber: Henkel AG & Co. KGaA, 40589 Düsseldorf (DE)
(72) Erfinder: GERKE, Thomas, 40627 Düsseldorf (DE); KROPF, Christian, 40724 Hilden (DE); HUCHEL, Ursula, 50670 Köln (DE); GRIESBECK, Axel, 50937 Köln (DE); HINZE, Olga, 50679 Köln (DE)
(86) Internationale Anmeldenummer: PCT/EP2011/050242
(87) Internationale Veröffentlichungsnummer: WO 2011/101179

(56) Entgegenhaltungen:
- WO-A1-2009/118219
- WO-A2-01/96272
- MAJEWSKI MAREK ET AL: "Reactions of .beta.-pinene with aromatic aldehydes", SYNTHETIC COMMUNICATIONS, TAYLOR & FRANCIS GROUP, PHILADELPHIA, PA, Bd. 20, Nr. 16, 1. Januar 1990 (1990-01-01), Seiten 2549-2558, XP009150532, ISSN: 0039-7911
- XIAO ZHUANQUAN ET AL: "Synthesis of endo-.alpha.-isocamphanyl ketones and .beta.-isocamphanyl alcohols", CHEMISTRY AND INDUSTRY OF FOREST PRODUCTS / LIN CHAN HUA XUE YU GONG YE, CHINESE ELECTRONIC PERIODICAL SERVICES, CHINA, Bd. 16, Nr. 3, 1. Januar 1996 (1996-01-01) , Seiten 19-23, XP008106157, ISSN: 0253-2417
- HERBERT CHARLES BROWN ET AL: "Reaction of B-alkyl-9-borabicyclo[3.3.1]nonanes with .alpha.-bromo ketones under the influence of potassium tert-butoxide. A convenient procedure for the .alpha.-alkylation of ketones", JOURNAL OF THE AMERICAN CHEMICAL SOCIETY, Bd. 91, Nr. 8, 1. April 1969 (1969-04-01), Seiten 2147-2149, XP55003361, ISSN: 0002-7863, DOI: 10.1021/ja01036a070
- C. WADE DOWNEY ET AL: "One-Pot Enol Silane Formation-Mukaiyama Aldol-Type Addition to Dimethyl Acetals Mediated by TMSOTf", THE JOURNAL OF ORGANIC CHEMISTRY, Bd. 73, Nr. 8, 1. April 2008 (2008-04-01), Seiten 3299-3302, XP55003348, ISSN: 0022-3263, DOI: 10.1021/jo8001084
- ZHENG G ET AL: "Lobelane analogues as novel ligands for the vesicular monoamine transporter-2", BIOORGANIC & MEDICINAL CHEMISTRY, PERGAMON, GB, Bd. 13, Nr. 12, 2. Juni 2005 (2005-06-02), Seiten 3899-3909, XP004903726, ISSN: 0968-0896, DOI: DOI:10.1016/J.BMC.2005.04.013
- C. KISHAN REDDY ET AL: "Neue Cobalt- und Eisen-katalysierte Reaktionen mit Organozinkverbindungen", ANGEWANDTE CHEMIE (INTERNATIONAL ED. IN ENGLISH), Bd. 108, Nr. 15, 2. August 1996 (1996-08-02), Seiten 1812-1813, XP55003330, ISSN: 0044-8249, DOI: 10.1002/ange.19961081523
- P. ZHONG ET AL: "New Synthetic Route to Ketones from Camphene and -Pinene", CHEMISTRY OF NATURAL COMPOUNDS, Bd. 39, Nr. 6, 1. November 2003 (2003-11-01), Seiten 549-552, XP55003334, ISSN: 0009-3130, DOI: 10.1023/B:CONC.0000018108.77705.0c
- DONALD D. PHILLIPS ET AL: JOURNAL OF THE AMERICAN CHEMICAL SOCIETY, Bd. 80, Nr. 6, 20. März 1958 (1958-03-20), Seiten 1360-1366, XP55003329, ISSN: 0002-7863, DOI: 10.1021/ja01539a022
- ORTIZ R ET AL: "Tandem intramolecular carbolithiation-transmetallation: from lithium to copper or boron chemistry", TETRAHEDRON, ELSEVIER SCIENCE PUBLISHERS, AMSTERDAM, NL, Bd. 61, Nr. 7, 14. Februar 2005 (2005-02-14), Seiten 1699-1707, XP025383021, ISSN: 0040-4020, DOI: DOI:10.1016/J.TET.2004.12.047 [gefunden am 2005-02-14]
- MALOSH C F ET AL: "Catalytic cross-coupling of alkylzinc halides with alpha-chloro ketones", JOURNAL OF THE AMERICAN CHEMICAL SOCIETY, AMERICAN CHEMICAL SOCIETY, WASHINGTON, DC; US, Bd. 126, Nr. 33, 1. Januar 2004 (2004-01-01), Seiten 10240-10241, XP003018893, ISSN: 0002-7863, DOI: DOI:10.1021/JA0467768
- V. V. ZHDANKIN ET AL: "Carbon-carbon bond formation in reactions of PhIO.cntdot.HBF4-silyl enol ether adduct with alkenes or silyl enol ethers", THE JOURNAL OF ORGANIC CHEMISTRY, Bd. 54, Nr. 11, 1. Mai 1989 (1989-05-01), Seiten 2605-2608, XP55003310, ISSN: 0022-3263, DOI: 10.1021/jo00272a028
- ROGER L. SNOWDEN ET AL: "Fragmentation of Homoallylic Alkoxides. Thermolysis of potassium 2-substituted bicyclo [2.2.2]oct-5-en-2-alkoxides", HELVETICA CHIMICA ACTA, Bd. 64, Nr. 7, 4. November 1981 (1981-11-04), Seiten 2193-2202, XP55003307, ISSN: 0018-019X, DOI: 10.1002/hlca.19810640726
- MALKOV A V ET AL: "Molybdenum(II)- and Tungsten(II)-Catalyzed Allylic Substitution", JOURNAL OF ORGANIC CHEMISTRY, AMERICAN CHEMICAL SOCIETY, EASTON.; US, Bd. 64, Nr. 8, 1. Januar 1999 (1999-01-01) , Seiten 2737-2750, XP002282721, ISSN: 0022-3263, DOI: DOI:10.1021/JO9821776
- IWASAWA NOBUHARU ET AL: "Synthesis of Medium-Sized Bicyclic Compounds by Intramolecular Cyclization of Cyclic .beta.-Keto Radicals Generated from Cyclopropanols Using Manganese(III) Tris(pyridine-2-carboxylate) and Its Application to Total Synthesis of 10-Isothiocyanatoguaia-6-ene", BULLETIN OF THE CHEMICAL SOCIETY OF JAPAN, CHEMICAL SOCIETY OF JAPAN, TOKYO, JP, Bd. 72, Nr. 1, 1. Januar 1999 (1999-01-01) , Seiten 85-97, XP002527309, ISSN: 0009-2673, DOI: DOI:10.1246/BCSJ.72.85
- AN PAI LI ET AL: "First Total Synthesis of an Analogue of (+-)-Hypargenin B", CHINESE CHEMICAL LETTERS, HTTP://WWW.IMM.AC.CN/JOURNAL/CCL.HTML, Bd. 13, Nr. 2, 1. Januar 2002 (2002-01-01) , Seiten 133-134, XP007919124, [gefunden am 2002-01-01]

## Beschreibung

Die Erfindung liegt auf dem Gebiet der Duftspeicherstoffe, wie sie zum Beispiel in Wasch- oder Reinigungsmitteln oder Kosmetika Verwendung finden. Die Erfindung betrifft insbesondere spezielle Ketone, die als photolabile Duftspeicherstoffe fungieren. Ferner betrifft die vorliegende Erfindung Wasch- oder Reinigungsmittel, kosmetische Mittel sowie Luftpflegemittel, welche solche Ketone enthalten. Ferner betrifft sie ein Verfahren zur lang anhaltenden Beduftung von Oberflächen und ebenso ein Verfahren zur lang anhaltenden Raumbeduftung. Sie betrifft ferner ein Verfahren zur Herstellung des genannten Ketons.

Wasch- und Reinigungsmittel bzw. kosmetische Mittel enthalten zumeist Duftstoffe, die den Mitteln einen angenehmen und frischen Geruch verleihen. Die Duftstoffe maskieren dabei zumeist die Eigenduftnote der anderen Inhaltstoffe, so dass beim Verbraucher ein positiver Geruchseindruck entsteht. Im Bereich Waschmittel sind Duftstoffe besonders wichtige Bestandteile der Zusammensetzung, da die Wäsche sowohl im feuchten als auch im trockenen Zustand einen angenehmen und frischen Duft aufweisen soll. Ein grundsätzliches Problem bei der Verwendung von Duftstoffen besteht darin, dass es sich bei diesen um flüchtige Substanzen handelt, ansonsten könnte kein Dufteffekt erzielt werden. Man steht daher bei dem Einsatz von Duftstoffen z.B. in Wasch- und Reinigungsmitteln ebenso wie bei dem Einsatz in kosmetischen Mitteln vor dem Problem, dass man der trotz der Flüchtigkeit der Verbindungen einen lange anhaltenden und möglichst gleichbleibenden Dufteffekt bewirken möchte. Hinzu kommt, dass sich der Dufteindruck eines Parfums im Laufe der Zeit verändert, weil die Riechstoffe, die die frischen und leichten Noten des Parfüms darstellen durch ihren hohen Dampfdruck schneller abdampfen als die Duftstoffe, die die Herz- und Basisnoten darstellen.

Ein Ansatz zur Lösung dieses Problems besteht darin, Duftstoffe auf Trägermaterialien aufzubringen und die bedufteten Träger zu beschichten, oder Duftstoffe zu verkapseln oder in Verbindungen einzulagern (beispielsweise Cyclodextrin-Parfüm-Komplexe). Des Weiteren existiert die Möglichkeit, die Duftstoffe chemisch an Trägermedien zu binden, wobei die chemische Bindung langsam gespalten und der Duftstoff hierdurch freigesetzt wird. Eine solche Träger-gebundene Vorform eines Duftstoffes wird auch als "Pro-Fragrance" oder Duftspeicherstoff bezeichnet.
In diesem Zusammenhang offenbart die internationale Patentanmeldung WO 2007/087977 die Verwendung von 1-Aza-3,7-dioxabicyclo[3.3.0]octan-Verbindungen als Duftspeicherstoffe zur verzögerten Freisetzung von Duftaldehyden und Duftketonen durch Hydrolyse. Eine alternative Möglichkeit der verzögerten Freisetzung von Duftstoffen stellt der Einsatz von so genannten photoaktivierbaren Substanzen als Duftspeicherstoffe dar. Durch die Einwirkung des Sonnenlichts oder einer anderen elektromagnetischen Strahlungsquelle bestimmter Wellenlänge wird der Bruch einer kovalenten Bindung im Duftspeicherstoff-Molekül induziert, wodurch ein Duftstoff freigesetzt wird. Der beschriebene Prozess muss dabei für eine wirksame Freisetzung des Duftstoffs die Anwesenheit von Sauerstoff und Wasser tolerieren.
In diesem Zusammenhang offenbart das US-Patent 6,949,680 die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität, die durch den photochemisch induzierten Zerfall erst verzögert und über einen längeren Zeitraum hinweg auf einer bestimmten Oberfläche freigesetzt wird. Dabei werden die genannten photolabilen Phenyl- oder Pyridylketone als Duftspeicherstoffe in einem aufwendigen, vielstufigen Syntheseverfahren unter Einsatz von Schutzgruppenoperationen hergestellt, wobei die Synthese für jeden einzelnen Aktivstoff individuell angepasst werden muss. In WO 2009/118219 A1 werden photoaktivierbare Substanzen als Duftspeicherstoffe beschrieben, welche die verzögerte Freisetzung von cyclischen Alkenen mit sogenannter semicyclischer Doppelbindung erlauben.
Ziel der vorliegenden Erfindung war die Bereitstellung photoaktivierbarer Substanzen als Duftspeicherstoffe, welche die verzögerte Freisetzung cyclischer Verbindungen mit mindestens einer cyclischen Doppelbindung, insbesondere cyclischer Terpene oder cyclischer Terpenoide mit mindestens einer cyclischen Doppelbindung erlauben. Ein weiteres Ziel der Erfindung war die Bereitstellung eines einfachen und kostengünstigen Syntheseverfahrens zur Herstellung der genannten Duftspeicherstoffe.

Überraschenderweise wurde gefunden, dass cyclische Phenylketone des vorliegenden Anspruchs 1 als photoaktivierbare Duftspeicherstoffe die verzögerte Freisetzung von cyclischen Verbindungen mit mindestens einer cyclischen Doppelbindung erlauben. Gegenstand der vorliegenden Erfindung ist daher ein Keton der allgemeinen Formel (I), 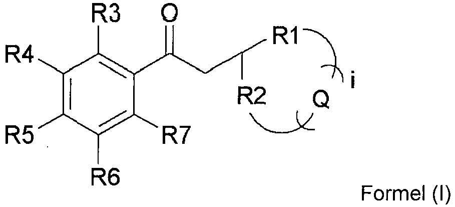
wobei i = 1 oder 2 ist,
R3, R4, R5, R6 und R7 unabhängig voneinander für Wasserstoff, ein Halogenatom, NO₂, eine lineare oder verzweigte Alkoxygruppe mit 1 bis 15 C-Atomen oder eine lineare oder verzweigte Alkylgruppe mit 1 bis 15 C-Atomen stehen und
Q, R1 und R2 so gewählt sind, dass durch Spaltung einer Kohlenstoffbindung ein Keton der allgemeinen Formel (II) 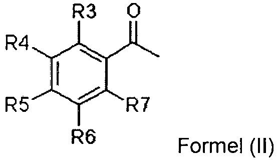 und ein Geruchsstoff aus der Klasse der cyclischen Terpene oder cyclischen Terpenoide mit mindestens einer cyclischen Doppelbindung der allgemeinen Formel (III) gebildet wird, 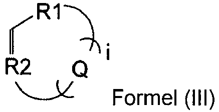 wobei der Geruchsstoff ausgewählt ist aus der Gruppe bestehend aus α-Pinen, α-Terpinen, Limonen, β-Bisabolen, Humulen, Terpinolen, Phellandren, Trichodien sowie aus deren Enantiomeren und/oder Diastereomeren.

Unter dem Begriff sekundäres C-Atom ist im Sinne der Erfindung ein Kohlenstoffatom zu verstehen, dass mit zwei weiteren Kohlenstoffatomen kovalent verbunden ist. Unter dem Begriff tertiäres C-Atom bzw. quartäres C-Atom ist im Sinne der Erfindung ein C-Atom zu verstehen, dass mit drei bzw. vier weiteren Kohlenstoffatomen kovalent verbunden ist.

Die erfindungsgemäßen Duftspeicherstoffe, erlauben die verzögerte Freisetzung cyclischer Verbindungen mit zumindest einer cyclischen Doppelbindung, bei denen es sich beispielsweise um die olfaktorisch bedeutende Klasse der cyclischen Terpene mit cyclischer Doppelbindung handelt. Diese stellen eine wichtige Klasse von Geruchsstoffen dar. Diese können beispielsweise dazu beitragen, Wasch- oder Reinigungsmitteln oder kosmetischen Mitteln einen angenehmen und frischen Geruch zu verleihen. Sie zeichnen sich in der Regel durch ihren hohen Dampfdruck aus und sind aufgrund ihres niedrigen Funktionalisierungsgrads chemisch nur schwer an herkömmliche Trägermedien zu binden. Die erfindungsgemäßen Duftspeicherstoffe vermögen es, die genannten cyclischen Verbindungen über einen längeren Zeitraum hinweg freizusetzen. Die Anwendung der erfindungsgemäßen Duftspeicherstoffe in Wasch-, Reinigungs- oder Pflegemitteln führte bei deren Anwendung zu einer verbesserte Langzeitduftwirkung, insbesondere im Zusammenhang mit der Textilbehandlung. Z.B. konnte bei der Anwendung erfindungsgemäßer Duftspeicherstoffe in einem Wäschebehandlungsmittels, wie z.B. Waschmittel sowie Weichspüler, eine verbesserte Langzeitduftwirkung der behandelten Wäsche gefunden werden. Ferner weisen entsprechende Produkte eine besonders gute Lagerstabilität auf. Die erfindungsgemäßen Mittel ermöglichen es zudem, die Gesamtmenge an Parfüm, welche im Mittel enthalten ist, zu reduzieren, und dennoch Geruchsvorteile auf den gewaschenen Textilien zu erzielen, insbesondere mit Blick auf das Frischeempfinden. Die langsame Freisetzung des gespeicherten Riechstoffes erfolgt nach Einwirkung von Licht umfassend die Wellenlängen von 200 bis 400 nm. In einer besonders bevorzugten Ausführungsform der Erfindung stehen R1 und R2 unabhängig voneinander für ein sekundäres oder tertiäres Kohlenstoffatom. In einer ganz besonders bevorzugten Ausführungsform der Erfindung steht einer der beiden Reste R1 und R2 für ein sekundäres Kohlenstoffatom, während der jeweils andere Rest für ein tertiäres Kohlenstoffatom steht.

Ein weiterer Gegenstand der vorliegenden Erfindung ist ein Wasch- oder Reinigungsmittel (wie vorzugsweise Textil- oder Oberflächenbehandlungsmittel, Textilwaschmittel, Weichspüler), enthaltend mindestens ein Keton der allgemeinen Formel (I) wie zuvor definiert, wobei das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,5 und 2 Gew.-%, jeweils bezogen auf das gesamte Mittel, enthalten ist.

Geeignete Reinigungsmittel sind z.B. Reinigungsmittel für harte Oberflächen, wie vorzugsweise Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken, insbesondere um einen sogenannten WC-Stein.

Ein kosmetisches Mittel kann mindestens ein Keton der allgemeinen Formel (I) wie zuvor definiert enthalten, wobei das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,5 und 2 Gew.-%, jeweils bezogen auf das gesamte Mittel, enthalten ist.

Ein Luftpflegemittel (z.B. Raumlufterfrischer, Raumdeodorant, Raumspray usw.) kann mindestens ein Keton der allgemeinen Formel (I) wie zuvor definiert enthalten, wobei das genannte Keton vorzugsweise in Mengen zwischen 0,0001 und 50 Gew.-%, vorteilhafterweise zwischen 0,01 und 5 Gew.-%, weiter vorteilhaft zwischen 0,1 und 3 Gew.-%, insbesondere zwischen 0,5 und 2 Gew.-%, jeweils bezogen auf das gesamte Mittel, enthalten ist.

Ein weiterer Gegenstand der vorliegenden Erfindung ist ein Verfahren zur lang anhaltenden Beduftung von Oberflächen (wie z.B. Textilien oder harte Oberflächen, Haut, Haar), wobei ein erfindungsgemäßes Keton der allgemeinen Formel (I) oder ein erfindungsgemäßes Mittel (wie z.B ein Wasch- oder Reinigungsmittel, ein kosmetisches Mittel), enthaltend ein erfindungsgemäßes Keton, auf die zu beduftende Oberfläche (wie z.B. Textil, harte Oberfläche, Haut, Haar) aufgebracht wird und die genannte Oberfläche anschließend einer elektromagnetischen Strahlung umfassend die Wellenlängen von 200 bis 400 nm ausgesetzt wird. Als elektromagnetische Strahlung im Sinne der vorliegenden Erfindung kann vorzugsweise das natürliche Sonnenlicht gelten.

Ebenfalls Gegenstand der vorliegenden Erfindung ist ein Verfahren zur Herstellung eines erfindungsgemäßen Ketons der allgemeinen Formel (I) umfassend folgende Schritte:
a) Hydroborierung eines Geruchsstoffs aus der Klasse der cyclischen Terpene oder cyclischen Terpenoide mit mindestens einer cyclischen Dooppelbindung, wobei der Geruchsstoff ausgewählt wird aus der Gruppe bestehend aus α-Pinen, α-Terpinen, Limonen, β-Bisabolen, Humulen, Terpinolen, Phellandren, Trichodien sowie aus deren Enantiomeren und/oder Diastereomeren,
b) Umsetzung des in Schritt a) erzeugten Organoboraddukts mit einer Verbindung der allgemeinen Formel (IV), 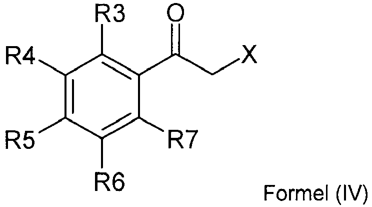 wobei X ein Halogenatom ist und R3, R4, R5, R6 und R7 unabhängig voneinander für Wasserstoff, ein Halogenatom, NO₂, eine lineare oder verzweigte Alkoxygruppe mit 1 bis 15 C-Atomen oder eine lineare oder verzweigte Alkylgruppe mit 1 bis 15 C-Atomen stehen.

Ein Keton der allgemeinen Formel (I) kann als Duftspeicherstoff verwendet werden und die Verwendung eines erfindungsgemäßen Ketons in Wasch- oder Reinigungsmitteln (wie insbesondere flüssigen oder festen Waschmitteln, Weichspülern, weichmachenden Waschmitteln, Waschhilfsmitteln) oder in kosmetischen Mitteln oder in Luftpflegemitteln.

Ebenfalls kann ein erfindungsgemäßes Keton, insbesondere enthalten in erfindungsgemäßen Mitteln wie z.B. Wasch- oder Reinigungsmittel oder kosmetisches Mittel, zur Verbesserung der Duftstoffausbeute verwendet werden, insbesondere auf Textilien, z.B. im Rahmen der maschinellen Textilwäsche.

Bevorzugt im Sinne der Erfindung ist ein erfindungsgemäßes Keton der allgemeinen Formel (I), in dem i = 2 ist und mindestens eine der R1 und R2 verbrückenden, zweiwertigen Gruppen Q 2 bis 6 C-Atome umfasst.

Besonders bevorzugt ist ein erfindungsgemäßes Ketone der allgemeinen Formel (I), in dem i = 2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst.

Ebenfalls bevorzugt ist ein erfindungsgemäßes Keton der allgemeinen Formel (I), in dem i = 2 ist und eine der R1 und R2 verbrückenden, zweiwertigen Gruppen Q verzweigt ist und die kürzeste direkte Verbindung zwischen R1 und R2 aus 2, 3 oder 4 jeweils kovalent miteinander verknüpften Kohlenstoffatomen gebildet wird. Ganz besonders bevorzugt im Sinne der Erfindung ist ein erfindungsgemäßes Keton der allgemeinen Formel (I), in dem i = 2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst, während die andere R1 und R2 verbrückende, zweiwertige Gruppen Q verzweigt ist und die kürzeste direkte Verbindung zwischen R1 und R2 aus 2, 3 oder 4 jeweils kovalent miteinander verknüpften Kohlenstoffatomen gebildet wird.

Weiterhin ist ein erfindungsgemäßes Keton der allgemeinen Formel (I) bevorzugt, in dem vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, insbesondere -F, -Cl oder -Br, NO₂, eine lineare oder verzweigte Alkoxygruppe mit 1 bis 15 C-Atomen oder eine lineare oder verzweigte Alkylgruppe mit 1 bis 15 C-Atomen steht. In einer überaus bevorzugten Ausführungsform der Erfindung steht R5 für -Cl, -Br, -NO₂ oder eine Alkyl- oder Alkoxygruppe umfassend 1 bis 4 C-Atome. Vorzugsweise handelt es sich bei der linearen oder verzweigten, substituierten oder unsubstituierten Alkylgruppe um eine Methyl- oder Ethylgruppe und/oder bei der linearen oder verzweigten. Alkoxygruppe um eine Methoxy-, Ethoxy-, Isopropoxy- oder tert-Butoxy-Gruppe.

Eine Substitution in para-Position (R5) ist besonders bevorzugt, da die elektronische Struktur des aromatischen Rings hier am effektivsten modifiziert werden kann, wodurch das Absorptionsmaximum von Ketonen der allgemeinen Formel (I) leicht an eine bestimmte Wellenlänge angepasst werden kann.

Ebenfalls bevorzugt ist ein erfindungsgemäßes Keton der allgemeinen Formel (I), in dem R3, R4, R5, R6 und R7 für Wasserstoff stehen.

Weiterhin im Sinne der Erfindung ist ein erfindungsgemäßes Keton der allgemeinen Formel (I), aus dem durch Spaltung einer Kohlenstoffbindung ein Keton der allgemeinen Formel (II) 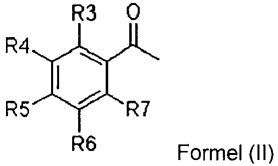 und eine Verbindung der allgemeinen Formel (III) gebildet wird, 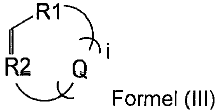 wobei der Geruchsstoff ausgewählt ist aus der Gruppe bestehend aus α-Pinen, α-Terpinen, Limonen, β-Bisabolen, Humulen, Terpinolen, Phellandren, Trichodien sowie aus deren Enantiomeren und/oder Diastereomeren.

In einer besonders bevorzugten Ausführungsform der Erfindung stehen R1 und R2 in Formel (III) unabhängig voneinander für ein sekundäres oder tertiäres Kohlenstoffatom. In einer ganz besonders bevorzugten Ausführungsform der Erfindung steht einer der beiden Reste R1 und R2 für ein sekundäres Kohlenstoffatom, während der jeweils andere Rest für ein tertiäres Kohlenstoffatom steht.

Bevorzugt ist eine erfindungsgemäße Verbindung der allgemeinen Formel (II), in der vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, NO₂, eine lineare oder verzweigte Alkoxygruppe mit 1 bis 15 C-Atomen oder eine lineare oder verzweigte Alkylgruppe mit 1 bis 15 C-Atomen steht. In einer überaus bevorzugten Ausführungsform der Erfindung steht R5 für -Cl, -Br, -NO₂ oder eine Alkyl- oder Alkoxygruppe umfassend 1 bis 4 C-Atome.

Vorzugsweise handelt es sich bei der linearen oder verzweigten Alkylgruppe um eine Methyl- oder Ethylgruppe und/oder bei der linearen oder verzweigten Alkoxygruppe um eine Methoxy-, Ethoxy-, Isopropoxy- oder tert-Butoxy-Gruppe.

Bevorzugt im Sinne der Erfindung ist ein erfindungsgemäßes Keton der allgemeinen Formel (I), aus dem durch Spaltung einer Kohlenstoffbindung eine Verbindung der allgemeinen Formel (III) hervorgeht, in der i = 2 ist und mindestens eine der R1 und R2 verbrückenden, zweiwertigen substituierten oder unsubstituierten Gruppen Q 2 bis 6 C-Atome umfasst.

Besonders bevorzugt ist dabei eine Verbindung der allgemeinen Formel (III), in der i = 2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst.

Ebenfalls bevorzugt ist eine Verbindung der allgemeinen Formel (III), in der i = 2 ist und eine der R1 und R2 verbrückenden, zweiwertigen Gruppen Q verzweigt ist und die kürzeste direkte Verbindung zwischen R1 und R2 aus 2, 3 oder 4 jeweils kovalent miteinander verknüpften Kohlenstoffatomen gebildet wird.

Ganz besonders bevorzugt im Sinne der Erfindung ist ein erfindungsgemäßes Keton der allgemeinen Formel (I), aus dem durch Spaltung einer Kohlenstoffbindung eine Verbindung der allgemeinen Formel (III) hervorgeht, in der i = 2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst, während die andere R1 und R2 verbrückende, zweiwertige Gruppen Q verzweigt ist und die kürzeste direkte Verbindung zwischen R1 und R2 aus 2, 3 oder 4 jeweils kovalent miteinander verknüpften Kohlenstoffatomen gebildet wird.

Eine Verbindung der allgemeinen Formel (III) ist ein cyclisches Terpen oder cyclisches Terpenoid mit mindestens einer cyclischen Doppelbindung.

Unter Terpenen sind erfindungsgemäß alle aus Isoprenbasiseinheiten aufgebauten Naturstoffe und Derivate zu verstehen.

Unter Terpenoiden sind erfindungsgemäß alle aus Isoprenbasiseinheiten aufgebauten Naturstoffe und Derivate zu verstehen, die eine hohe strukturelle Verwandtschaft zu den Terpenen aufweisen, sich von diesen aber beispielsweise durch den Verlust oder die Umlagerung eines Fragments, vorzugsweise einer Methylgruppe unterscheiden.

Je nach Zahl der Basiseinheiten teilt man die Terpene in Monoterpene, Sesquiterpene, Diterpene, Sesterpene, Triterpene und Tetraterpene ein.

Bevorzugt sind gemäß einer bevorzugten Ausführungsform insbesondere monocyclische Monoterpene, bicyclische Monoterpene und cyclische Sesquiterpene. Alle erfindungsgemäßen Terpene umfassen dabei mindestens eine cyclische Doppelbindung.

Die cyclischen Terpene oder cyclischen Terpenoide mit cyclischer Doppelbindung werden ausgewählt aus der Gruppe bestehend aus α-Pinen, α-Terpinen, Limonen, β-Bisabolen, Humulen, Terpinolen, Phellandren, Trichodien sowie aus deren Enantiomeren und/oder Diastereomeren.

Die genannten cyclischen Terpene oder cyclischen Terpenoide zeichnen sich in der Regel durch eine hohe Isomerisierungstendenz aus. Bei langer Sonnenlichtexposition kann es zu Gerüstumlagerungen (z.B. durch Wagner-Meerwein-Umlagerungen) oder zur Bildung von Doppelbindungsisomeren kommen. Dies ist unerwünscht, da die genannten Isomere sich oft deutlich in ihrem Geruchseindruck von den ursprünglich vorhandenen cyclischen Terpenen oder cyclischen Terpenoiden unterscheiden. Unter Umständen kann so der Dufteindruck einer Mehrkomponenten-Parfümölmischung entscheidend verändert werden. Ein Vorteil der vorliegenden Erfindung ist daher die Tatsache, dass die erfindungsgemäßen Terpene oder Terpenoide erst direkt bei der Anwendung durch Sonnenlichtexposition freigesetzt werden, wodurch eine Duftveränderung durch vorhergehende Isomerisierungsreaktionen nahezu ausgeschlossen werden kann.

Gegenstand der vorliegenden Erfindung ist ebenfalls ein Mittel (wie insbesondere ein Wasch- oder Reinigungsmittel), enthaltend mindestens eine Verbindung der allgemeinen Formel (la), 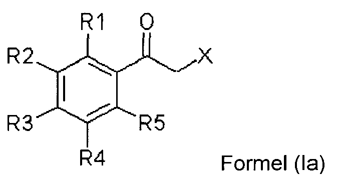 wobei R1, R2, R3, R4 und R5 unabhängig voneinander für Wasserstoff, ein Halogenatom, NO₂, eine lineare oder verzweigte Alkoxygruppe mit 1 bis 15 C-Atomen oder eine lineare oder verzweigte Alkylgruppe mit 1 bis 15 C-Atomen stehen,
und X nach Spaltung der C-X-Bindung für eine zyklische Verbindung, enthaltend mindestens eine cyclische Doppelbindung steht.

Bei dem Rest X handelt es sich nach Spaltung der C-X-Bindung um ein cyclisches Terpen mit cyclischer Doppelbindung, ausgewählt aus der Gruppe bestehend aus α-Pinen, α-Terpinen, Limonen, β-Bisabolen, Humulen, Terpinolen, Phellandren, Trichodien sowie aus deren Enantiomeren und/oder Diastereomeren.

Bevorzugt ist eine erfindungsgemäße Verbindung der allgemeinen Formel (la), in der vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, NO2, eine lineare oder verzweigte Alkoxygruppe mit 1 bis 15 C-Ato-men oder eine lineare oder verzweigte Alkylgruppe mit 1 bis 15 C-Atomen steht. In einer überaus bevorzugten Ausführungsform der Erfindung steht R5 für -Cl, -Br, -NO2 oder eine Alkyl- oder Alkoxygruppe umfassend 1 bis 4 C-Atome. Vorzugsweise handelt es sich bei der linearen oder verzweigten

Alkylgruppe um eine Methyl- oder Ethylgruppe und/oder bei der linearen oder verzweigten Alkoxygruppe um eine Methoxy-, Ethoxy-, Isopropoxy- o-der tert-Butoxy-Gruppe.

In einer bevorzugten Ausführungsform der Erfindung enthält ein erfindungsgemäßes Mittel, wie z.B. Wasch- oder Reinigungsmittel mindestens einen weiteren Duftstoff. Die bevorzugt eingesetzten Duftstoffe bzw. Parfümöle sind keinerlei Beschränkungen unterworfen. So können vorzugsweise als Duftstoffe synthetische oder natürliche Riechstoffverbindungen vom Typ der Ester, Ether, Aldehyde (Duftaldehyde), Ketone (Duftketone), Alkohole, Kohlenwasserstoffe, Säuren, Kohlensäureester, aromatischen Kohlenwasserstoffe, aliphatischen Kohlenwasserstoffe, gesättigten und / oder ungesättigten Kohlenwasserstoffe und Mischungen daraus verwendet werden.

Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann allgemein bekannt. Die Duftketone können alle Ketone umfassen, die einen erwünschten Duft oder ein Frischeempfinden verleihen können. Es können auch Gemische unterschiedlicher Ketone eingesetzt werden. Einsetzbare Ketone sind z.B. Alpha Damascon, Delta Damascon, Iso Damascon, Carvon, Gamma-Methylionon, Iso-E-Super, 2,4,4,7-Tetramethyl-oct-6-en-3-on, Benzylaceton, Beta Damascon, Damascenon, Methyldihydrojasmonat, Methylcedrylon, Hedion und Gemischen davon. Geeignete Duftaldehyde können beliebige Aldehyde sein, die entsprechend der Duftketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Lilial usw. Die Duftaldehyde und Duftketone eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen. Für weitere geeignete Duftstoffe, ausgewählt aus Aldehyden und Ketonen, wird auf Steffen Arctander Published 1960 and 1969 respectively, Reprinted 2000 ISBN: Aroma Chemicals Vol. 1: 0-931710-37-5, Aroma Chemicals Vol. 2: 0-931710-38-3, verwiesen. Geeignete Duftstoffe vom Typ der Ester sind beispielsweise Benzylacetat, Phenoxyethylisobutyrat, p-tert.-Butylcyclohexylacetat usw. Riechstoffverbindungen vom Typ der Kohlenwasserstoffen sind z.B. Terpene wie Limonen und Pinen. Geeignete Duftstoffe vom Typ Ether sind beispielsweise Benzylethylether und Ambroxan. Geeignete Duftstoffalkohole sind beispielsweise 10-Undecen-1-ol, 2,6-Dimethylheptan-2-ol, 2-Methylbutanol, 2-Methylpentanol, 2-Phenoxyethanol, 2-Phenylpropanol usw. Duftstoffe bzw. Parfümöle können auch natürliche Riechstoffgemische sein, wie sie aus pflanzlichen Quellen zugänglich sind. Bei den Duftstoffen bzw. Parfümölen kann es sich auch um ätherische Öle, wie z.B. Angelikawurzelöl, Anisöl, Arnikablütenöl usw. handeln. Die Gesamtmenge des mindestens einen weiteren Duftstoffs in dem erfindungsgemäßen Mittel, wie z.B. Wasch- oder Reinigungsmittel oder kosmetisches Mittel, beträgt vorzugsweise zwischen 0,001 und 5 Gew.-%, vorteilhafterweise zwischen 0,01 und 4 Gew.-%, weiter vorteilhaft zwischen 0,1 und 3 Gew.-% sowie ganz besonders bevorzugt zwischen 0,5 und 2 Gew.-% bezogen auf das gesamte Mittel. Bevorzugt werden Mischungen verschiedener Duftstoffe (aus den verschiedenen oben genannten Duftstoffklassen) verwendet, die gemeinsam eine ansprechende Duftnote erzeugen.

In einer bevorzugten Ausführungsform der Erfindung enthalten erfindungsgemäße Mittel (wie. z.B. Wasch- oder Reinigungsmittel) mindestens ein Tensid, vorzugsweise ausgewählt aus der Gruppe bestehend aus anionischen, kationischen, nichtionischen, zwitterionischen, amphoteren Tensiden oder deren Mischungen.

Ein erfindungsgemäßes Mittel kann vorzugsweise fest oder flüssig sein, wobei flüssige Mittel, insbesondere Waschmittel, bevorzugt sind. Insbesondere für den Fall, dass das erfindungsgemäße Mittel ein Waschmittel ist, ist es bevorzugt, dass es mindestens ein Tensid ausgewählt aus der Gruppe bestehend aus anionischen, nichtionischen, zwitterionischen und amphoteren Tensiden enthält. Insbesondere für den Fall, dass das erfindungsgemäße Mittel ein weichmachendes Waschmittel ("2in1") ist, ist es bevorzugt, dass es eine weichmachende Komponente sowie mindestens ein Tensid ausgewählt aus der Gruppe bestehend aus anionischen, nichtionischen, zwitterionischen und amphoteren Tensiden enthält. Waschhilfsmittel werden zur gezielten Vorbehandlung der Wäsche vor dem Waschen bei Flecken oder starker Verschmutzung eingesetzt. Zu den Waschhilfsmitteln gehören beispielsweise Vorbehandlungsmittel, Einweichmittel, Entfärber und Fleckensalz.

Insbesondere für den Fall, dass das erfindungsgemäße Mittel ein Weichspüler ist, ist es bevorzugt, dass es eine weichmachende Komponente enthält. Weichspüler sind als erfindungsgemäße Mittel bevorzugt, da sie erst im letzten Schritt eines konventionellen Textilwaschvorgangs, dem Spülgang, in Kontakt mit den Textilien kommen und somit eine möglichst große Menge der Duftstoffe auf das Textil aufziehen kann, ohne dass die Gefahr besteht, dass die Duftstoffe bei anschließen-den Schritten wieder entfernt werden. Es ist ganz besonders bevorzugt, dass die weichmachende Komponente eine alkylierte, quaternäre Ammoniumverbindung ist, wobei mindestens eine Alkylkette durch eine Ester- oder Amidogruppe unterbrochen ist. Die weichmachende Komponente umfasst beispielsweise quaternäre Ammoniumverbindungen wie Monoalk(en)yltrimethylammonium-Verbindungen, Dialk(en)yldimethylammonium-Verbindungen, Mono-, Di- oder Triester von Fettsäuren mit Alkanolaminen. Weitere erfindungsgemäß verwendbare weichmachende Komponenten stellen quaternisierten Proteinhydrolysate oder protonierte Amine dar. Weiterhin sind auch kationische Polymere geeignete weichmachende Komponente. Ebenfalls einsetzbar sind polyquaternierte Polymere (z.B. Luviquat® Care von BASF) und auch kationische Biopolymere auf Chitinbasis und deren Derivate, beispielsweise das unter der Handelsbezeichnung Chitosan® (Hersteller: Cognis) erhältliche Polymer. Weitere geeignete weichmachende Komponenten umfassen protonierte oder quaternierte Polyamine. Besonders bevorzugte weichmachende Komponenten sind alkylierte quaternäre Ammoniumverbindungen, von denen mindestens eine Alkylkette durch eine Estergruppe und/oder Amidogruppe unterbrochen ist. Ganz besonders bevorzugt sind N-Methyl-N-(2-hydroxy-ethyl)-N,N-(ditalgacyloxyethyl)ammonium-methosulfat oder Bis-(palmitoyloxyethyl)-hydroxyethyl-methyl-ammonium-methosulfat.

Das erfindungsgemäße Mittel, insbesondere in Form von Weichspülern, kann auch nichtionische weichmachende Komponenten enthalten, wie vor allem Polyoxyalkylenglycerolalkanoate, Polybutylene, langkettige Fettsäuren, ethoxylierte Fettsäureethanolamide, Alkylpolyglucoside, insbesondere Sorbitanmono,-di- und -triester, und Fettsäureester von Polycarbonsäuren. In dem erfindungsgemäßen Weichspüler als erfindungsgemäßen Mittel ist vorteilhafterweise die weichmachende Komponente in Mengen von 0,1 bis 80 Gew.-%, üblicherweise 1 bis 40 Gew.-%, vorzugsweise 2 bis 20 Gew.-% und insbesondere 3 bis 15 Gew.-% und der mindestens eine Duftstoff oder die Mischung verschiedener Duftstoffe in Mengen von vorteilhafterweise 0,1 bis 20 Gew.-%, vorzugsweise 1 bis 13 Gew.-% und insbesondere 2 bis 8 Gew.-%, jeweils bezogen auf die Gesamtmenge des erfindungsgemäßen Mittels, enthalten.

Als weitere Komponente kann das erfindungsgemäße Mittel, insbesondere in Form eines Weichspülers, gegebenenfalls ein oder mehrere nichtionische Tenside enthalten, wobei solche eingesetzt werden können, die üblicherweise auch in Waschmitteln verwendet werden.

Es ist weiterhin bevorzugt, dass das erfindungsgemäße Mittel, insbesondere in Form eines Wasch- oder Reinigungsmittels, zusätzlich weitere vorteilhafte Inhaltsstoffe enthält, die dem Fachmann bekannt sind. So kann das erfindungsgemäße Mittel, wie insbesondere Wasch- oder Reinigungsmittel, zusätzlich zu den Tensiden und/oder weichmachenden Verbindungen weitere Inhaltsstoffe enthalten, die die anwendungstechnischen und/oder ästhetischen Eigenschaften des Mittels weiter verbessern. Im Rahmen der vorliegenden Erfindung enthalten bevorzugte Mittel zusätzlich einen oder mehrere Stoffe aus der Gruppe der Gerüststoffe, Bleichmittel, Bleichaktivatoren, Enzyme, Elektrolyte, nichtwässrigen Lösungsmittel, pH-Stellmittel, Parfüme, Parfümträger, Fluoreszenzmittel, Farbstoffe, Hydrotope, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber. Als Gerüststoffe, die in den erfindungsgemäßen Mitteln enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Als Reinigungsmittel kann das erfindungsgemäße Mittel z.B. zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Als harte Oberflächen kommen neben Geschirr auch alle übrigen harten Oberflächen, insbesondere aus Glas, Keramik, Kunststoff oder Metall, in Haushalt und Gewerbe in Frage. Dabei kann es sich, wie auch bei allen anderen erfindungsgemäßen Mitteln, um feste oder flüssige Formulierungen handeln, wobei feste Formulierungen als Pulver, Granulat, Extrudat, in Tab-Form, als Tablette oder als gepresster und/oder geschmolzener Formkörper vorliegen können. Bei flüssigen Formulierungen kann es sich um Lösungen, Emulsionen, Dispersionen Suspensionen, Mikroemulsionen, Gels oder Pasten handeln.

Die Herstellung fester erfindungsgemäßer Mittel (d.h. Wasch- oder Reinigungsmittel) bereitet keine Schwierigkeiten und kann im Prinzip bekannter Weise, zum Beispiel durch Sprühtrocknen oder Granulation, erfolgen, wobei optionale Persauerstoffverbindung und optionaler Bleichkatalysator gegebenenfalls später zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen. Die Einarbeitung der erfindungsgemäßen Duftspeicherstoffe kann insbesondere zusammen mit anderen Riechstoffen erfolgen.

Weiter oben wurde als weiterer Gegenstand der Erfindung bereits das Verfahren zur Herstellung eines erfindungsgemäßen Ketons der allgemeinen Formel (I) genannt, umfassend als Schritt a) die Hydroborierung und als Schritt b) die Umsetzung des in Schritt a) erzeugten Organoboraddukts mit einer Verbindung der allgemeinen Formel (IV), 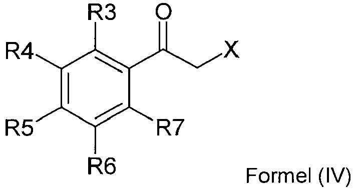 wobei X ein Halogenatom ist und R3, R4, R5, R6 und R7 unabhängig voneinander für Wasserstoff, ein Halogenatom, NO2, eine lineare oder verzweigte Alkoxygruppe mit 1 bis 15 C-Atomen oder eine lineare oder verzweigte Alkylgruppe mit 1 bis 15 C-Atomen stehen.

In einer besonders bevorzugten Ausführungsform des genannten Verfahrens steht X in einer Verbindung der allgemeinen Formel (IV) für -Cl oder -Br, insbesondere für -Br. In einer ebenfalls bevorzugten Ausführungsform des genannten Verfahrens ist eine Verbindung der allgemeinen Formel (IV) bevorzugt, in der vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, NO2, eine lineare oder verzweigte

Alkoxygruppe mit 1 bis 15 C-Atomen oder eine lineare oder verzweigte Alkylgruppe mit 1 bis 15 C-Atomen steht. In einer überaus bevorzugten Ausführungsform der Erfindung steht R5 für -Cl, -Br, - NO2 oder eine Alkyl- oder Alkoxygruppe umfassend 1 bis 4 C-Atome. Vorzugsweise handelt es sich bei der linearen oder verzweigten Alkylgruppe um eine Methyl- oder Ethylgruppe und/oder bei der linearen oder verzweigten Alkoxygruppe um eine Methoxy-, Ethoxy-, Isopropoxy- oder tert-Butoxy-Gruppe. Unter dem Begriff Hydroborierung ist im Sinne der Erfindung eine 1,2-Addition eines Organoboranreagenzes an zumindest eine cyclische Doppelbindung der Verbindung der allgemeinen Formel (III) zu verstehen, wodurch eine kovalente Kohlenstoff-Bor-Bindung und damit ein Organoboraddukt gebildet wird. Geeignete Verfahren zur Hydroborierung sind in J. March, Advanced Organic Chemistry, 4. Aufl., S. 783-789 beschrieben, worauf hier Bezug genommen wird.

Vorzugsweise führt man die Hydroborierung in einem Lösungsmittel durch. Geeignete Lösungsmittel für die Hydroborierung sind beispielsweise acyclische Ether wie Diethylether, Methyl-tert.-butylether, Dimethoxyethan, Diethylenglykoldimethylether, Triethylenglykoldimethylether, cyclische Ether wie Tetrahydrofuran oder Dioxan sowie Kohlenwasserstoffe wie Hexan oder Toluol oder Gemische davon. Die Reaktionstemperatur wird in der Regel von der Reaktivität des Hydroborierungsmittels bestimmt und liegt vorzugsweise zwischen dem Schmelz -und Siedepunkt des Reaktionsgemisches. Insbesondere kann die Reaktion in THF und/oder Diethylether bei Temperaturen zwischen -100 °C und 40 °C, vorzugsweise zwischen -40 °C und 30 °C und insbesondere zwischen 0 °C und 20 °C durchgeführt werden. Geeignete Organoboranreagenzien sind vorzugsweise sterisch anspruchsvolle Organoboranreagenzien, wobei diese beispielsweise ausgewählt werden können aus der Gruppe bestehend aus 9-Borabicyclo[3.3.1]nonan (9-BBN), 1,1,2-Trimethylpropylboran (Thexylboran), Catecholboran oder Diisopinocampheylboran (Ipc2BH). Vorzugsweise setzt man das Organoboranreagenz äquimolar oder im Überschuß bezogen auf die mindestens eine cyclische Doppelbindung der Verbindung der allgemeinen Formel (III) ein.

Ein Vorteil des genannten Verfahrens liegt in der Tatsache, dass die gebildeten Organoboraddukte vorzugsweise nicht isoliert werden müssen, sondern ohne vorhergehende Aufreinigung direkt mit einer Verbindung der allgemeinen Formel (IV) umgesetzt werden können. Die genannte Umsetzung des Organoboranaddukts mit der Verbindung der allgemeinen Formel (IV) erfolgt vorzugsweise in einem geeigneten Lösungsmittel, beispielsweise in acyclischen Ethern wie Diethylether, Methyl-tert.-butylether, Dimethoxyethan, Diethylenglykoldimethylether, Triethylenglykoldimethylether, in cyclischen Ethern wie Tetrahydrofuran oder in Dioxan sowie in Kohlenwasserstoffen wie Hexan oder Toluol oder in Gemischen davon, wobei die beschriebene Umsetzung überaus bevorzugt in Gegenwart eines Alkalimetallalkoholats durchgeführt wird. Die Reaktionstemperatur wird in der Regel von der Reaktivität der Verbindung der allgemeinen Formel (IV) und/oder von der Reaktivität des Organoboraddukts bestimmt und liegt vorzugsweise zwischen dem Schmelz -und Siedepunkt des Reaktionsgemisches. Insbesondere kann die Reaktion in THF und/oder Diethylether bei Temperaturen zwischen -100 °C und 40 °C, vorzugsweise zwischen -90 °C und 0 °C und insbesondere zwischen -78 °C und -40 °C durchgeführt werden.

In einer überaus bevorzugten Ausführungsform wird das genannte Alkalimetallalkoholat ausgewählt aus Kalium-methanolat, Natrium-methanolat, Kalium-ethanolat, Natrium-ethanolat, Kaliumpropylat, Natrium-propylat, Kalium-tert-butanolat, Natrium-tert-butanolat, Kalium-2,6-di-tert-butyl-phenolat, Natrium-2,6-di-tert-butylphenolat und aus deren beliebigen Mischungen. Vorzugsweise setzt man das Alkalimetallalkoholat äquimolar oder im leichten Überschuss (1,05 bis 1,30 eq.) bezogen auf das gebildete Organoboranaddukt ein.

Die Aufreinigung des gebildeten Ketons der allgemeinen Formel (I) erfolgt vorzugsweise durch Kristallisation, Destillation und/oder Säulenchromatographie. Ein Vorteil des beschriebenen Herstellungsverfahrens liegt in der Tatsache, dass die Ketone der allgemeinen Formel (I) in der Regel in einer einstufigen konvergenten Synthese ohne aufwendige Schutzgruppenoperationen direkt und kostengünstig hergestellt werden können. Es entsteht somit ein Duftspeicherstoff AB, der im Wesentlichen aus einem Photorezeptor (A) und einer cyclischen Verbindung mit cyclischer Doppelbindung (B) zusammengesetzt ist. Durch Bestrahlung zerfällt der genannte Duftspeicherstoff AB dann wieder problemlos in seine einzelnen Bestandteile A und B.

Ein bevorzugtes erfindungsgemäßes festes, insbesondere pulverförmiges Waschmittel kann neben dem erfindungsgemäßen Keton (gemäß Formel (I)) insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-%, vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
- Stabilisatoren, z.B. Phosphonate, vorteilhafterweise 0-1 Gew.-%
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibitor, z.B. Polyvinylpyrrolidon-Derivate, vorzugsweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. weitere Duftstoffe
- ggf. Wasser
- ggf.Seife
- ggf.Bleichaktivatoren
- ggf.Cellulosderivate
- ggf.Schmutzabweiser,
- Gew.-% jeweils bezogen auf das gesamte Mittel.

In einer anderen bevorzugten Ausführungsform der Erfindung liegt das Mittel in flüssiger Form vor, vorzugsweise in Gelform. Bevorzugte flüssige Mittel, wie insbesondere Wasch- oder Reinigungsmittel sowie Kosmetika, haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäßes flüssiges, insbesondere gelförmiges Waschmittel kann neben dem erfindungsgemäßen Keton (gemäß Formel (I)) insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Duftstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein bevorzugter erfindungsgemäßer flüssiger Weichspüler kann neben dem erfindungsgemäßen Keton (gemäß Formel (I)) insbesondere noch Komponenten enthalten, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. weitere Duftstoffe
- Farbstoffe, vorzugsweise im ppm-Bereich
- Stabilisatoren, vorzugsweise im ppm-Bereich
- Lösemittel, wie insbesondere Wasser, in Mengen von vorzugsweise 60-90 Gew.-%,
- Gew.-% jeweils bezogen auf das gesamte Mittel.

### Beispiele

### Beispiel 1: Darstellung eines Ketons der allg. Formel (I)

### Synthese von 3-(6, 6-Dimethylbicyclo [3.1.1]-heptan-2-yl)-1-phenylpropan-1-on

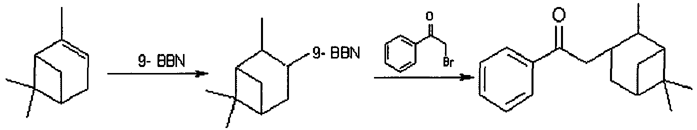

1.36 g (-)-α-Pinen (10 mmol) wurden in 5 ml THF gelöst. Zu dieser Lösung wurden 21 ml einer 0.5 molaren Lösung von 9-BBN (9-Borabicyclo[3.3.1]nonan) in THF (10.5 mmol) bei 0 °C zugefügt. Die Reaktionsmischung wurde langsam auf Raumtemperatur gebracht und weitere 3 Stunden gerührt. Die entstandene Lösung wurde auf -78 °C abgekühlt und anschließend eine Lösung von 1.12 g Kalium-tert-butanolat (10 mmol) in 10 ml THF zu der abgekühlten Reaktionslösung zugegeben. Nach kurzer Zeit wurden 2 g α-Bromacetophenon (10 mmol) portionsweise bei konstantem Rühren hinzu gegeben. Die Reaktionsmischung wurde langsam auf Raumtemperatur gebracht und 4 Stunden bei Raumtemperatur gerührt. Das entstandene Produktgemisch wurde mit 50 ml n-Pentan versetzt, 3-mal mit jeweils 10 ml einer 3 N Natriumhydroxidlösung und mit 10 ml Wasser gewaschen. Die organische Phase wurde über Magnesiumsulfat getrocknet. Das Filtrat wurde bei vermindertem Druck von Lösungsmittel befreit. Das Rohprodukt wurde säulenchromatographisch gereinigt. Es wurde 140 mg (10 %) des oben genannten Produkts erhalten.

### Beispiel 2 : Belichtung des Reaktionsproduktes aus Beispiel 1

20 mg des Reaktionsproduktes aus Beispiel 1 wurden in 8 ml Methanol gelöst. Die Reaktionslösung wurde in einem Multilamp-Photoreactor (8 W-Lampen (4x), Firma Luxchem) mit Emissionmaximum λ = 350 nm eine Stunde lang belichtet. Die Reaktion wurde mit Hilfe der GC/MS-Spektrometrie verfolgt. Nach spätestens 60 Minuten Belichtung wurde die weitgehend vollständige Umwandlung des genannten Reaktionsproduktes in Acetophenon und α-Pinen beobachtet.

### Beispiel 3: Riechtest

Für den im Folgenden beschriebenen Riechtest wurden 0,2 mmol des Reaktionsproduktes aus Beispiel 1 in 1 ml Acteon gelöst. In die Lösung wurde ein Riechstreifen 2 cm tief eingetaucht, der anschließend unter Lichtausschluss bei 20 °C getrocknet wurde.

Zum Vergleich wurden Lösungen von 0,1 mmol alpha-Pinen und 0.1 mmol Acetophenon in je 1 ml Aceton und eine Mischung von 0.1 mmol alpha-Pinen und 0.1 mmol Acetophenon in 1 ml Aceton hergestellt. Anschließend wurde in jede Lösung ein Riechstreifen 2 cm tief eingetaucht, wobei jeder Riechstreifen anschließend unter Lichtausschluss bei 20 °C getrocknet wurde.
Nach erfolgreicher Trocknung wird jeder Riechstreifen über den gesamten Testzeitraum hinweg mit einer handelsüblichen Leuchtstoffröhre (gemäß DIN 5035 neutralweiß (nw); Farbtemperatur 3300 bis 5500 K) bestrahlt und die Duftintensität zu den jeweils angegebenen Zeitpunkten bestimmt.

Die Duftintensität wurde von 3 trainierten Probanden auf einer Skala von 0 bis 6 bewertet, wobei 6 die höchste Note ist und 0 für keine Duftwahrnehmung steht.

### Definition der Skalierung:

- 6: unangenehm stark
- 5: sehr stark
- 4: stark
- 3: intensiv
- 2: angenehm
- 1: wahrnehmbar
- 0: nicht mehr wahrnehmbar

Die Ergebnisse der Riechtests sind in der folgenden Tabelle dargestellt, wobei die angegebenen Werte den Bereich der Duftwahrnehmung der Probandengruppe widerspiegeln.

| | nach 1 Minute | nach 20 Minuten | nach 45 Minuten | nach 1,5 Stunden | nach 24 Stunden |
|---|---|---|---|---|---|
| Photocage Pinen | chemischer Geruch stark nach Acetophenon 1 | 0-1 | 0-1 | 0 wird durch Tageslichlampe erneut riechbar 1 | 0 wird durch Tageslichlampe erneut riechbar 1 |
| Acetophenon | 3 | 3 | 2-3 | 2 | 0 |
| alpha-Pinen | 0-1 | 0 | 0 | 0 | 0 |
| Acetophenon/ alpha-Pinen | nur Acetophenon wahrnehmbar 2-3 | 2-3 | 2 | 2 | 0 |

Es zeigt sich, dass die ca. 1-minütige Bestrahlung mit einer 20 Watt Tageslichtlampe (LifeLite Full Spectrum Daylight Lamp) auch noch nach 24 Stunden die photoinduzierte Freisetzung des Geruchsstoffs alpha-Pinen bewirkt.

## Patentansprüche

1. Keton der allgemeinen Formel (I), 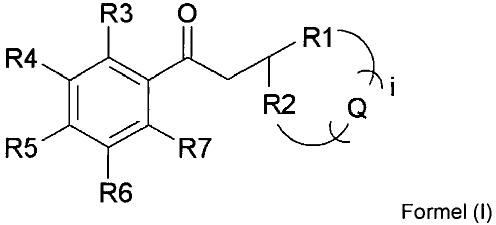
wobei i = 1 oder 2 ist,
R3, R4, R5, R6 und R7 unabhängig voneinander für Wasserstoff, ein Halogenatom, NO₂, eine lineare oder verzweigte Alkoxygruppe mit 1 bis 15 C-Atomen oder eine lineare oder verzweigte Alkylgruppe mit 1 bis 15 C-Atomen stehen und
Q, R1 und R2 so gewählt sind, dass durch Spaltung einer Kohlenstoffbindung ein Keton der allgemeinen Formel (II) 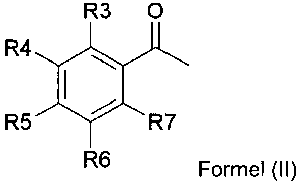 und ein Geruchsstoff aus der Klasse der cyclischen Terpene oder cyclischen Terpenoide mit mindestens einer cyclischen Doppelbindung der allgemeinen Formel (III) gebildet wird 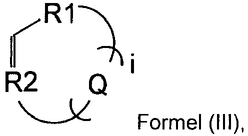 wobei der Geruchsstoff ausgewählt ist aus der Gruppe bestehend aus α-Pinen, α-Terpinen, Limonen, β-Bisabolen, Humulen, Terpinolen, Phellandren, Trichodien sowie aus deren Enantiomeren und/oder Diastereomeren.

2. Wasch- oder Reinigungsmittel enthaltend mindestens ein Keton nach Anspruch 1, **dadurch gekennzeichnet, dass** das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,5 und 2 Gew.-%, jeweils bezogen auf das gesamte Mittel, enthalten ist.

3. Verfahren zur lang anhaltenden Beduftung von Oberflächen, **dadurch gekennzeichnet, dass** ein Keton nach Anspruch 1 oder ein Mittel nach Anspruch 2 auf die zu beduftende Oberfläche aufgebracht wird und die genannte Oberfläche anschließend einer elektromagnetischen Strahlung umfassend die Wellenlängen von 200 bis 400 nm ausgesetzt wird.

4. Verfahren zur Herstellung eines Ketons der allgemeinen Formel (I) gemäß Anspruch 1 umfassend folgende Schritte:
a) Hydroborierung eines Geruchsstoffs aus der Klasse der cyclischen Terpene oder cyclischen Terpenoide mit mindestens einer cyclischen Doppelbindung, wobei der Geruchsstoff ausgewählt wird aus der Gruppe bestehend aus α-Pinen, α-Terpinen, Limonen, β-Bisabolen, Humulen, Terpinolen, Phellandren, Trichodien sowie aus deren Enantiomeren und/oder Diastereomeren,
b) Umsetzung des in Schritt a) erzeugten Organoboraddukts mit einer Verbindung der allgemeinen Formel (IV), 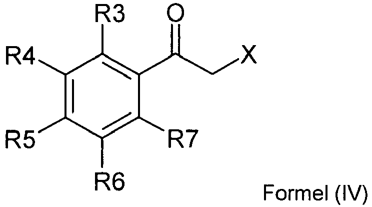 wobei X ein Halogenatom ist und R3, R4, R5, R6 und R7 unabhängig voneinander für Wasserstoff, ein Halogenatom, NO2, eine lineare oder verzweigte Alkoxygruppe mit 1 bis 15 C-Atomen oder eine lineare oder verzweigte Alkylgruppe mit 1 bis 15 C-Atomen stehen.

## Claims

1. A ketone of the general formula (I) 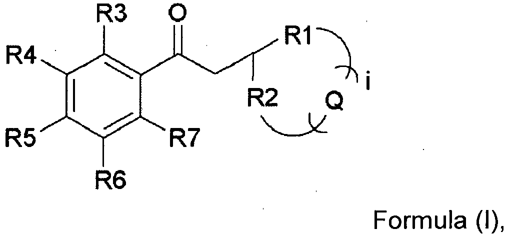
where i = 1 or 2,
R3, R4, R5, R6, and R7 mutually independently denote hydrogen, a halogen atom, NO₂, a linear or branched alkoxy group having 1 to 15 carbon atoms, or a linear or branched alkyl group having 1 to 15 carbon atoms, and
Q, R1 and R2 are selected so that by cleavage of a carbon bond, a ketone of the general formula (II) 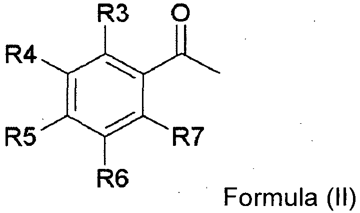 and an odour compound from the class of the cyclic terpenes or cyclic terpenoids having at least one cyclic double bond, of the general formula (III) 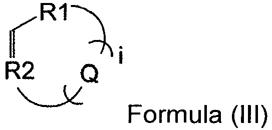 are formed, the odour compound being selected from the group consisting of α-pinene, α-terpinene, limonene, β-bisabolene, humulene, terpinolene, phellandrene, trichodiene, and from enantiomers and/or diastereomers thereof.

2. A washing or cleaning agent containing at least one ketone according to Claim 1, **characterised in that** said ketone is contained by preference in quantities between 0.0001 and 5 wt%, advantageously between 0.001 and 4 wt%, more advantageously between 0.01 and 3 wt%, in particular between 0.5 and 2 wt%, based in each case on the total agent.

3. A method for long-lasting scenting of surfaces, **characterised in that** a ketone according to Claim 1 or an agent according to Claim 2 is applied onto the surface to be scented, and said surface is then exposed to an electromagnetic radiation comprising the wavelengths from 200 to 400 nm.

4. A method for manufacturing a ketone of the general formula (I) in accordance with Claim 1, comprising the following steps:
a) hydroboration of an odour compound from the class of the cyclic terpenes or cyclic terpenoids having at least one cyclic double bond, the odour compound being selected from the group consisting of α-pinene, α-terpinene, limonene, β-bisabolene, humulene, terpinolene, phellandrene, trichodiene, and from enantiomers and/or diastereomers thereof.
b) reacting the organoborane adduct produced in step a) with a compound of the general formula (IV) 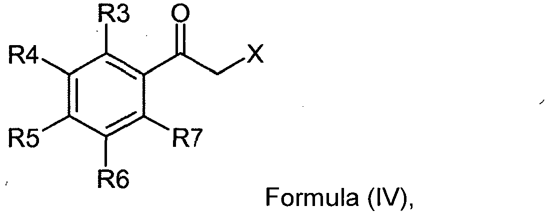 where X is a halogen atom and R3, R4, R5, R6, and R7 mutually independently denote hydrogen, a halogen atom, NO₂, a linear or branched alkoxy group having 1 to 15 carbon atoms, or a linear or branched alkyl group having 1 to 15 carbon atoms.

## Revendications

1. Cétone de la Formule générale (I), 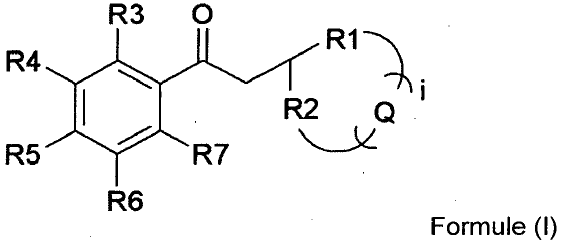
dans laquelle i est = 1 ou 2,
R3, R4, R5, R6 et R7 représentent indépendamment les uns des autres l'hydrogène, un atome d'halogène, NO₂, un groupe alcoxy linéaire ou ramifié comprenant de 1 à 15 atomes de C ou un groupe alkyle linéaire ou ramifié comprenant de 1 à 15 atomes de C et
Q, R1 et R2 sont sélectionnés de sorte que, par clivage d'une liaison carbone, une cétone de la Formule générale (II) 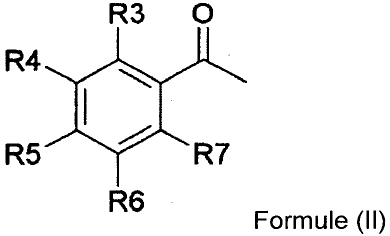 et une matière odorante de la classe des terpènes cycliques ou des terpénoïdes cycliques comprenant au moins une double liaison cyclique de la Formule générale (III) 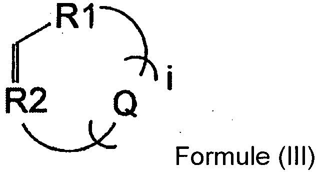 soient formées,
dans laquelle la matière odorante est sélectionnée dans le groupe constitué de l'α-pinène, de l'α-terpinène, du limonène, du ß-bisabolène, de l'humulène, du terpinolène, du phellandrène, du trichodiène ainsi que de leurs énantiomères et/ou diastéréomères.

2. Agent de lavage ou de nettoyage contenant au moins une cétone selon la revendication 1, **caractérisé en ce que** ladite cétone est contenue de préférence dans des quantités comprises entre 0,0001 et 5 % en poids, de manière avantageuse entre 0,001 et 4 % en poids, de manière encore plus avantageuse entre 0,01 et 3 % en poids, en particulier entre 0,5 et 2 % en poids, rapporté respectivement à l'agent total.

3. Procédé pour le parfumage longue durée de surfaces, **caractérisé en ce qu'**une cétone selon la revendication 1 ou un agent selon la revendication 2 est appliqué(e) sur la surface à parfumer et ladite surface est ensuite exposée à un rayonnement électromagnétique comprenant les longueurs d'onde de 200 à 400 nm.

4. Procédé pour la préparation d'une cétone de la Formule générale (I) selon la revendication 1 comprenant les étapes suivantes :
a) hydroboration d'une matière odorante de la classe des terpènes cycliques ou des terpénoïdes cycliques comprenant au moins une double liaison cyclique, dans lequel la matière odorante est sélectionnée dans le groupe constitué de l'α-pinène, de l'α-terpinène, du limonène, du ß-bisabolène, de l'humulène, du terpinolène, du phellandrène, du trichodiène ainsi que de leurs énantiomères et/ou diastéréomères,
b) réaction de l'adduit d'organobore produit à l'étape a) avec un composé de la Formule générale (IV), 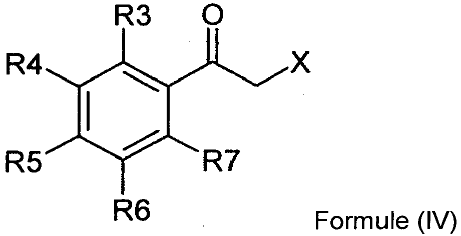 dans laquelle X est un atome d'halogène et R3, R4, R5, R6 et R7 représentent indépendamment les uns des autres l'hydrogène, un atome d'halogène, NO₂, un groupe alcoxy linéaire ou ramifié comprenant de 1 à 15 atomes de C ou un groupe alkyle linéaire ou ramifié comprenant de 1 à 15 atomes de C.
